# EUROPÄISCHE PATENTANMELDUNG

(11) **EP 0 085 023 A1**
(43) Veröffentlichungstag der Anmeldung: **03.08.1983**
(21) Anmeldenummer: 83810009.7
(22) Anmeldetag: 10.01.1983
(51) Int. Cl.: C07B 19/00, C07C 69/70, C07C 69/708

(54) **Verfahren zur Enantiomeren-Trennung durch Verteilung zwischen flüssigen Phasen**

(30) Priorität: 14.01.1982 CH 256/82
(71) Anmelder: CIBA-GEIGY AG, 4002 Basel (CH)
(72) Erfinder: Prelog, Vladimir, Prof., CH-8092 Zürich (CH)

(57) **Zusammenfassung**

Es wird ein Verfahren zur Enantiomeren-Trennung beschrieben, indem man zwischen einer wässrigen Phase und einer lipophilen Phase, die eines der beiden optisch aktiven Isomeren eines Weinsäureesters der Formel I 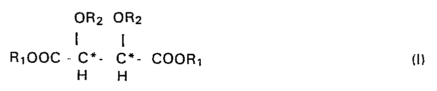 enthält oder aus diesem besteht, ein Enantiomerengemisch einer chiralen Verbindung verteilt, das mindestens teilweise aus einer Verbindung der Formel II 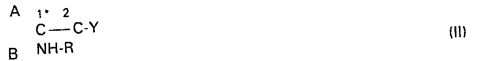 in Form eines Ammoniumsalzes mit einem lipophilen Anion besteht. Dabei haben A, B, R, Y, R₁ und R₂ die im Patentanspruch 1 angegebene Bedeutung. Im allgemeinen enthält die lipophile Phase neben dem Weinsäureester (I) auch noch ein mit Wasser nicht mischbares organisches Lösungsmittel. Bei diesem Trennverfahren geht das eine der beiden Enantiomeren der chiralen Verbindung in einem höheren Ausmass von der wässrigen Phase in die lipophile Phase über, wodurch in einfacher Weise die Enantiomeren-Trennung erreicht wird. Der verwendbare Weinsäureester (I) bleibt unverändert, kann wiederholt gebraucht werden und ist praktisch vollständig rückgewinnbar.

## Beschreibung

Die vorliegende Erfindung betrifft ein Verfahren zur Enantiomeren- Trennung zwischen flüssigen Phasen, d.h. einer wässrigen und einer lipophilen Phase.

Die Aufspaltung von Enantiomerengemischen, beispielsweise Racematen, in die beiden Enantiomeren ist ein bei der Durchführung von Herstellungsverfahren häufig auftretendes Problem, vor allem deshalb, weil beispielsweise auf dem Gebiete der Arzneimittel und Pestizide oft nur eines der beiden Enantiomeren die gewünschte Wirksamkeit zeigt, während das andere Enantiomer weniger wirksam bzw. unwirksam ist oder unerwünschte Nebenwirkungen zeigt.

Verteilungen zwischen einer flüssigen wässrigen Phase und einer flüssigen lipophilen Phase werden in der chemischen Technik in grossem Massstab durchgeführt, beispielsweise im Zusammenhang mit der Reinigung von synthetischen Produkten oder der Isolierung von Naturstoffen. Deshalb sind Verfahren, die auf solchen Verteilungen beruhen, besonders wertvoll. Optisch aktive flüssige Phasen, z.B. Kohlenwasserstoffe, wie Pinen, oder Alkohole, wie optisch aktiver 2-Methyl-2-butanol oder Octanol-2, sind jedoch im allgemeinen nicht enantiomerenselektiv.

Es ist zwar bekannt, dass sich gewisse chirale Kronenäther zur Enantiomeren-Trennung durch Verteilung von Ammoniumsalzen von Entantiomerengemischen zwischen einer wässrigen und einer lipophilen Phase eignen. Die dabei in der lipophilen Phase eingesetzten chiralen Kronenäther sind jedoch teuer und schwer zugänglich. Sie sind ferner vom toxikologischen Standpunkt und wegen ihrer ausgeprägten komplexbildenden Eigenschaften auch vom ökologischen Standpunkt aus nicht unbedenklich [vgl. z.B. Cram et al., JACS, 95:8,2692(1973), 95:9,3021(1973), 96:21,6762(1974), 96:22,7100(1974), 96:23,7367(1974) und 100:14,4555(1978) sowie J. Org. Chem., 42,4173(1977)].

Gegenstand der Erfindung ist ein einfaches, besonders wirtschaftliches Verfahren zur Enantiomeren-Trennung durch Verteilung zwischen flüssigen Phasen, bei dem leicht zugängliche enantiomere lipophile Verbindungen eingesetzt werden können, die im Gegensatz zu den oben erwähnten Kronenäthern von Cram et al. toxikologisch unbedenklich sind und in technischen Verfahren keine nennenswerte Belastung der Umwelt zur Folge haben. Das erfindungsgemässe Verfahren ist dadurch gekennzeichnet, dass man zwischen einer wässrigen Phase und einer lipophilen Phase, welche eines der beiden optisch aktiven Isomeren eines Weinsäureesters der Formel I 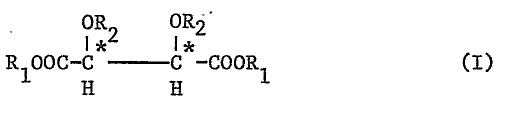 enthält oder aus diesem besteht, ein Enantiomerengemisch einer chiralen Verbindung verteilt, das mindestens teilweise aus einer Verbindung der Formel II 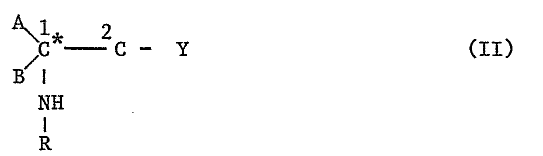 in Form eines Ammoniumsalzes mit einem lipophilen Anion besteht, worin
A und B Wasserstoffatome oder organische Reste sind oder
A und B gemeinsam mit dem Kohlenstoffatom, an das sie gebunden sind, eine Ringstruktur darstellen, wobei jedoch A und B bzw, die an das ¹C-Atom gebundenen Gruppierungen oder Atome voneinander verschieden sein müssen, so dass die Verbindung chiral ist,
R ein Wasserstoffatom oder einen organischen Rest darstellt,
Y ein Sauerstoff-, Stickstoff- oder Schwefelatom bedeutet, welches die Ausbildung einer Wasserstoffbrücke ermöglicht, wobei Y über eine Einfach-, Zweifach- oder Dreifachbindung an das Kohlenstoffatom gebunden ist und Y und/oder das Kohlenstoffatom, an welches Y gebunden ist, gegebenenfalls durch Wasserstoff oder organische Reste substituiert sein können, die R₁ gleiche oder verschiedene organische Reste und die R unabhängig voneinander Wasserstoff oder einen organischen Rest bedeuten, wobei das eine der beiden Enantiomeren der zu trennenden chiralen Verbindung in einer grösseren Menge aus der wässrigen Phase in die lipophile Phase übergeht als das andere und somit eine Enantiomerenanreicherung oder Enantiomerentrennung erreicht wird.

Das erfindungsgemäss zu verwendende System zeigt trotz der im Vergleich zu den bekannten chiralen Kronenäthern relativ geringen Stabilität der durch die Ester der Formel I gebildeten diastereomeren Komplexe ein überraschend hohes enantiomeren-selektives Verhalten. Die Weinsäureester der Formel I können mehrfach verwendet oder z.B. durch Destillation praktisch vollständig zurückgewonnen und für weitere Enantiomeren-Trennungen eingesetzt werden.

Die Weinsäureester der Formel I sind technisch leicht zugänglich. Ein weiterer Vorteil des erfindungsgemässen Verfahrens besteht darin, dass die genannten Weinsäureester sowohl in der R,R- als auch der S,S-Konfiguration eingesetzt werden können. Unter Umständen kann es von Vorteil sein, die Weinsäureester abwechselnd in der R,R- und S,S-Konfiguration zu verwenden, beispielsweise in einem iterativen Verfahren.

Das erfindungsgemäss einzusetzende Enantiomerengemisch muss definitionsgemäss zwischen einer wässrigen und einer lipophilen Phase verteilbar sein und mindestens teilweise eine Verbindung der Formel II in Form eines Ammoniumsalzes mit einem lipophilen Anion aufweisen. Dabei kann im Enantiomerengemisch der Anteil an Verbindungen der FormelII in Form von Ammoniumsalzen mit einem lipophilen Anion je nach Art des Anions variieren.

Die lipophile Phase kann ausschliesslich aus dem optisch aktiven Weinsäureester der Formel I bestehen. Im allgemeinen ist es jedoch zweckmässig, eine Lösung des optisch aktiven Weinsäureesters in einem mit Wasser nicht mischbaren organischen Lösungsmittel als lipophile Phase einzusetzen, wobei das Lösungsmittel selbst bevorzugt nicht zur Ausbildung von Wasserstoffbrücken befähigt ist. Geeignete organische Lösungsmittel für die lipophile Phase sind z.B. Kohlenwasserstoffe und halogenierte Kohlenwasserstoffe, vor allem chlorierte aliphatische Kohlenwasserstoffe, wie Trichlorfluormethan und vor allem Chloroform Tetrachlorkohlenstoff, Trichloräthan und 1,2-Dichloräthan, 1,2-Dichloräthan ist besonders bevorzugt.

Als organische Reste stellen A oder B z.B. geradkettiges oder verzweigtes Alkyl mit 1-10 und vor allem 1-4 C-Atomen, das durch NH₂-, OH-, SH-, COOH-, CONH₂- oder -S-C₁₋₄-Alkylgruppen oder durch eine NH2- und eine OH-Gruppe substituiert sein kann, 1,3- oder 1,4-Cyclohexadienyl oder Phenyl oder Benzyl, die durch eine oder zwei OH-oder Methoxygruppen substituiert sein können, dar. Besonders bevorzugt weisen Alkylgruppen A oder B 1 oder 2 C-Atome auf. Als Beispiele von gegebenenfalls definitionsgemäss substituierten Alkylgruppen A oder B seien gennant: Methyl, Aethyl, n-Propyl, Isopropyl, n-Butyl, Isobutyl, 2-Butyl, n-Pentyl, n-Hexyl, 2-Aethylhexyl, n-Heptyl, n-Octyl, n-Nonyl und n-Decyl; Hydroxymethyl, 1-Hydroxyäthyl, Mercaptomethyl, 2-Mercaptoäthyl, 2-Methylthioäthyl, Aminocarbonylmethyl, 2-Aminocarbonyläthyl, Carboxymethyl, 2-Carboxyäthyl, 3-Aminopropyl, 4-Aminobutyl und 4-Amino-2-hydroxybutyl. Sind Phenyl-oder Benzylgruppen A oder B durch OH- oder Methoxygruppen substituiert, so handelt es sich z.B. um die 3,4-Dihydroxyphenyl-, 3-oder 4-Hydroxyphenyl-, 3,4-Dimethoxyphenyl-, 3- oder 4-Hydroxybenzyl-, 3,4-Dihydroxybenzyl- oder 3,4-Dimethoxybenzylgruppe.

Bilden A und B zusammen mit dem C-Bindungsatom eine Ringstruktur, so kommen z.B.Cyclohexyl-1-, 2-Phenylcyclopentyl-1- oder 3-Methyl-cyclopentyl-1-Gruppen in Betracht.

Enthalten die Reste A oder B weitere Aminogruppen, so können auch diese mindestens teilweise in Form von Ammoniumsalzen mit lipophilen Anionen vorliegen.

Vorzugsweise stellt eines von A und B Wasserstoff und das andere C₁₋₄-Alkyl, C₁₋₄-Hydroxyalkyl oder Phenyl, vor allem Methyl, Hydroxymethyl oder Phenyl, dar.

Stellt R einen organischen Rest dar, so handelt es sich z.B. um Phenyl, das durch eine oder zwei C₁₋₄-Alkylgruppen substituiert sein kann und insbesondere um geradkettiges oder verzweigtes Alkyl mit 1-6 C-Atomen, wie Methyl, Aethyl, n-Propyl, Isopropyl, n-Butyl, Isobutyl, n-Pentyl und n-Hexyl. R ist vorzugsweise Methyl und insbesondere Wasserstoff.

Die R₁ sind bevorzugt aliphatische oder cycloaliphatische Reste, besonders Alkyl- oder Alkenylgruppen mit mindestens 4 C-Atomen oder Cycloalkyl- oder Cycloalkenylgruppen mit mindestens 4 C-Atomen, die durch C₁₋₄-Alkylgruppen substituiert sein können. Die genannten Alkyl- oder Alkenylgruppen R können geradkettig oder verzweigt sein. Bei den Cycloalkyl- oder Cycloalkenylgruppen P₁ kann es sich auch um polycyclische (überbrückte) Systeme handeln.

Als Alkylgruppen R₁ werden solche mit 6-20 C-Atomen, besonders verzweigte Alkylgruppen mit 6-20 und vor allem 6-12 C-Atomen bevorzugt. Geeignete derartige Alkylgruppen sind z.B. die 2-Hexyl-, 1-Heptyl-, 4-Heptyl-, 1-Octyl-, 2R-Octyl-, 2S-Octyl-, 1-Nonyl, 2-Nonyl-, 3-Nonyl-, 4-Nonyl-, 5-Nonyl-, 1-Undecyl-, 2-Undecyl-, 6-Undecyl-, 2-Dodecyl- und 1-Tetradecylgruppe.

Bedeutet R₁ eine Alkenylgruppe so handelt es sich bevorzugt um Alkenylgruppen mit 4-12 C-Atomen, vor allem die 10-Undecenylgruppe.

Als Cycloalkyl- oder Cycloalkenylgruppen R ₁kommen vor allem solche mit insgesamt 6-20 C-Atomen in Betracht. Bevorzugt sind mono- oder bicyclische Cycloalkenyl- und vor allem Cycloalkylgruppen R₁ mit insgesamt 6-20 C-Atomen. Als Beispiele seien genannt: die Cyclohexyl-, Cycloheptyl-, Cyclooctyl-, Cyclodecyl-, Cyclododecyl-, Methylcyclohexyl-, Cyclohexenyl-, Cycloheptenyl-, Menthyl-, Neomenthyl-, 2-Bornyl-, Isobornyl- und Norbornylgruppe.

Als organische Reste R₂ kommen z.B. Acetyl-, Benzoyl-, Toluoyl- und vor allem geradkettige oder verzweigte Alkylgruppen mit 1-6 C-Atomen und insbesondere die Methylgruppe in Betracht. Besonders bevorzugt stellen die R₂ je Methyl und vor allem je Wasserstoff dar.

Vorzugsweise werden als Verbindungen der Formel I Ester von Alkoholen oder Alkoholgemischen verwendet, die bei der Anwendungstemperatur nicht leicht kristallisieren, da ein Auskristallisieren der Weinsäureester in der lipophilen Phase unerwünscht ist. Es können auch Gemische verschiedener Verbindungen der Formel I verwendet werden.

Weinsäureester der Formel I, die sich besonders gut zur Durchführung des erfindungsgemässen Verfahrens eignen, sind die Ester der folgenden Alkohole: 1- und 4-Heptanol, Cycloheptanol, 1-Octanol,2-Octanol, 1, 2-, 3-, 4- und 5-Nonanol, Borneol, 2- und 6-Undecanol sowie Mischungen dieser Alkohole. Die genannten Weinsäureester mit relativ lipophiler Alkoholkomponente erweisen sich gegenüber weniger lipophilen, im Handel erhältlichen Weinsäureestern, wie zum Beispiel dem Diäthyl-(R,R)-tartrat und dem Di-n-butyl-(R,R)-tartrat, als eindeutig überlegen.

Besonders bevorzugt verwendet man Verbindungen der Formel I, worin die R₂ je Methyl und insbesondere je Wasserstoff und die R Alkylgruppen mit 6-20, besonders 6-12 C-Atomen, vor allem derartige verzweigte Alkylgruppen, oder gegebenenfalls durch eine Methylgruppe substituierte mono- oder bicyclische Cycloalkylgruppen mit insgesamt 6-20, besonders 6-12 C-Atomen darstellen.

Besonders bevorzugt verwendet man Verbindungen der Formel I, worin die R₂ je Wasserstoff sind und die R₁ je die Cycloheptyl- oder die 2-Bornylgruppe und vor allem je die 5-Nonylgruppe bedeuten.

Als Beispiele von geeigneten Gruppen -C-Y seien erwähnt: 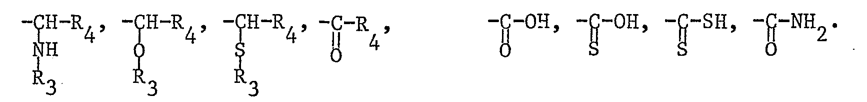 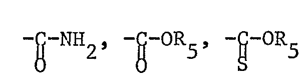 und -C≡N, worin R₃ Wasserstoff, Acetyl, Benzoyl oder geradkettiges oder verzweigtes Alkyl, besonders C₁₋₆-Alkyl und vor allem Methyl ist, R₄ Wasserstoff, Phenyl oder geradkettiges oder verzweigtes Cₗ₋₆-Alkyl und R₅ Benzyl oder geradkettiges oder verzweigtes C1-6-Alkyl darstellen.

Enthalten Gruppierungen -C-Y NH₂-Gruppen, so können auch diese mindestens teilweise in Form von Ammoniumsalzen mit lipophilen Anionen vorliegen.

R₃ ist vorzugsweise Methyl und insbesondere Wasserstoff, R₄ stellt bevorzugt Wasserstoff, Methyl oder Phenyl dar und R₅ ist insbesondere ^{C}ₗ₋₄-Alkyl, vor allem Methyl.

Bevorzugte zu trennende chirale Verbindungen sind 1,2-Diamine und vor allem a-Aminoalkohole, a-Aminosäuren, a-Aminosäureester und a-Aminosäureamide. Besonders bevorzugt stellt -C-Y eine der folgenden Gruppierungen dar: 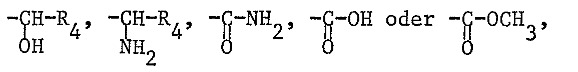 wobei R₄ Wasserstoff, Methyl oder Phenyl ist.

Mit Hilfe des erfindungsgemässen Verfahrens können z.B. die folgenden Enantiomerengemische getrennt werden: Ephedrin, Pseudo-Ephedrin, Norephedrin, Pseudo-Norephedrin, erythro-2-Amino-1,2-diphenyläthanol, threo-2-Amino-1,2-diphenyläthanol, erythro- oder threo-l-Amino-l-phenyl-2-propanol, Phenylglycinol, Phenylglycin, Phenylglycinmethylester, Phenylglycinamid, threo-2-Amino-1-phenyl-1,3-propandiol oder threo-1,2-Diamino-1,2-diphenyläthan. Bevorzugt verwendet man als Enantiomerengemische Norephedrin, Ephedrin, Pseudo-Ephedrin, threo-2-Amino-l-phenyl-l,3-propandiol, Phenylglycin, Phenylglycinmethylester oder Phenylglycinamid.

Bei der Durchführung des erfindungsgemässen Verfahrens setzt man zweckmässig Enantiomerengemische in Form von Ammoniumsalzen mit hydrophilem Anion, z.B. als Chloride, ein, die bei der Trennung mindestens teilweise gegen lipophile Anionen ausgetauscht werden. Geeignete lipophile Anionen sind z.B. das Hexafluorphosphat, Hexafluorsilikat, Hexafluorantimonat, Hexafluorarsenat, Tetraphenylborat, Tetrafluorborat, Pikrat, Perchlorat, Rhodanid und Jodid. Bevorzugt ist das lipophile Anion das Hexafluorphosphat, Tetraphenylborat oder Tetrafluorborat. Besonders gute Ergebnisse werden unter Verwendung von Hexafluorphosphat erzielt.

Das lipophile Anion und der Anteil an Verbindungen der Formel I im Enantiomerengemisch müssen so gewählt werden, dass das Enantiomerengemisch weder vollständig in der wässrigen Phase verbleibt, noch vollständig in die lipophile Phase übergeht, weil sonst keine Enantiomerenanreicherung möglich ist. Wird beispielsweise bei den geprüften Aminoalkoholen pro vorhandenes Ammoniumion ein Aequivalent Tetraphenylborat eingesetzt, so gehen diese Aminoalkohole wegen der starken Lipophilie des Anions vollständig in die lipophile Phase über. Wird hingegen nur ein halbes Aequivalent Tetraphenylborat pro vorhandenes Ammoniumion zugegeben, so erreicht man eine beträchtliche Enantiomerentrennung.

Das erfindungsgemässe Verfahren kann durch einmalige Verteilung oder bevorzugt durch iterative Verteilung durchgeführt werden. Wegen ihrer starken Lipophilie eignen sich z.B. die Tetraphenylborsalze enantiomerer Ammoniumionen weniger gut für iterative Verfahren. Für iterative Verfahren können z.B. verschiedene an sich bekannte Apparaturen für technische Mehrstufenextraktionen, Chromatographiesäulen der in den Beispielen 4 und 5 beschriebenen Art oder Craig-Apparaturen verwendet werden.

Analytische Versuche zeigen, dass es für die Trennung der Enantiomerengemische am günstigsten ist, wenn die Mengen des Ammoniumsalzes in der wässrigen Phase und in der lipophilen Phase ungefähr gleich gross sind. Dies kann erreicht werden, indem man entweder die Konzentration des Weinsäureesters der Formel I entsprechend hoch wählt, oder indem man bei niedrigeren Konzentrationen das Volumen der lipophilen Phase entsprechend erhöht. Bei den in den Beispielen beschriebenen Versuchen arbeitet man mit einer Erhöhung des Volumens der lipophilen Phase.

Bei der Durchführung des erfindungsgemässen Verfahrens kann die Enantiomerenselektivität im allgemeinen durch eine Temperaturerniedrigung beträchtlich gesteigert werden. Das erfindungsgemässe Verfahren wird daher zweckmässig bei einer Temperatur durchgeführt, die oberhalb des Erstarrungspunktes der wässrigen Phase bzw. der liophilen Phase liegt, vorzugsweise im Bereich von -5 bis +5°C, besonders 0 bis +5°C.

Die Erfindung wird anhand der Beispiele näher erläutert.

Beispiel 1: Herstellung des Di-(5-nonyl)-(R,R)-tartrates.

45,0 g (0,3 Mol) (+)-Weinsäure werden mit 108,2 g (0,75 Mol) 5-Nonanol in 600 ml Toluol unter Zusatz von 3,6 g Methansulfonsäure unter Rückfluss verestert. Nach dem azeotropen Ueberdestillieren der erwarteten Menge Wasser wird das erkaltete Reaktionsgemisch mit gesättigten Lösungen von Natriumhydrogencarbonat und Kochsalz sowie mit Wasser gewaschen. Durch Destillation der über Magnesiumsulfat getrockneten Toluol-Lösung werden 103,5 g farbloses Di-(5-nonyl)-(R,R)-tartrat, Spd. 150°C_{/}1,3 Pa, 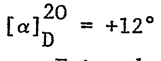 (c = 1,6 %, Aceton) erhalten. In Tabelle I sind weitere Ester der Formel I und deren Eigenschaften aufgeführt, die auf analoge Weise hergestellt werden.

### Beispiel 2: Herstellung des Dimethyläthers des Di-(5-nonyl)-(R,R)-tartrates.

Eine Lösung von 40,5 g Di-(5-nonyl)-(R,R)-tartrat und 24,8 g Dimethylsulfat in 50 ml Diäthyläther wird im Verlaufe von 1 Std. unter mechanischem Rühren zu einer Suspension von 12 g Natriumhydrid (hergestellt aus einer 50%igen Suspension von NaH in Paraffinöl, durch Waschen mit Diäthyläther) in 400 ml Diäthyläther zugetropft, wobei der Diäthyläther langsam siedet. Nachher wird über Nacht weitergerührt und vom Niederschlag abfiltriert. Dieser wird gründlich mit Diäthyläther nachgewaschen. Die vereinigten Aether-Lösungen werden mit gesättigten Natriumhydrogencarbonat- und Kochsalz-Lösungen sowie mit Wasser gewaschen, mit Magnesiumsulfat getrocknet und im Vakuum destilliert. Man erhält 44,8 g des Dimethyläthers des Di-(5-nonyl)-(R,R)-tartrates in Form eines farblosen Oels; Sdp. 150°C/2,7 Pa; 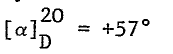 (c = 1,8 %, Aceton, k_{S}= 3,3; k_{S}/k"_{S} = 1,27) [k_{S}, k'/k" = Werte unter Standard-Bedingungen].

Die geeigneten Bedingungen zur Enantiomeren-Trennung lassen sich aus zwei Grössen ableiten: a) aus den Verteilungskoeffizienten k der zu trennenden Enantiomerengemische zwischen den verwendeten wässrigen und lipophilen Phasen und b) aus dem Verhältnis k'/k" des im Ueberschuss vorliegenden Enantiomeren zu dem im Unterschuss vorhandenen Enantiomeren in beiden Phasen:

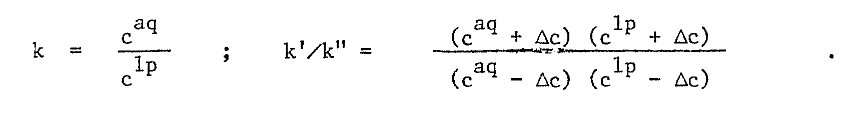

Diese Grössen werden auf analytischem Wege bestimmt, wobei c^{aq} die Konzentration des Enantiomerengemisches in der wässrigen Phase und c^{lp} die Konzentration des Enantiomerengemisches in der lipophilen Phase ist. Ac ist die Konzentration des Ueberschusses an einem der Enantiomeren.

Diese Grössen, die von der Konzentration des verwendeten Esters und des lipophilen Anions, der Art des lipophilen Lösungsmittels und von der Temperatur abhängig sind, werden für die in Tabelle I aufgeführten Ester unter Standard-Bedingungen (0,1 Mol Ester in 1,2-Dichloräthan, 0,5 Mol Natriumhexafluorphosphat und 0,05 Mol Norephedrin-hydrochlorid in Wasser bei 4°C) gemessen. Die analogen, unter den angegebenen Bedingungen gemessenen Grössen k und k'/k" sind in Tabelle II für verschiedene Enantiomerengemische zusammengestellt.

### Beispiel 3: Enantiomerentrennung durch einmalige Verteilung:

In einem mit einem Rührer und einem Kühlmantel versehenen zylindrischen Scheidetrichter werden 20 ml einer 0,215-molaren Lösung von (+)-Norephedrin-hydrochlorid (808 mg) in 2,0-molarer Natriumhexafluorphosphat-Lösung mit 100 ml einer 0,37-molaren Lösung von Di-(5-nonyl)-(R,R)-tartrat in 1,2-Dichloräthan 2 Std. bei 0°C gerührt. In der wässrigen Phase werden 386 mg Norephedrin-hydrochlorid mit 14,7 Gew.-% (R)-Enantiomerenüberschuss gefunden. Die lipophile Phase wird zuerst mit 0,25 N Natriumhydroxidlösung und dann mit 0,1 N Salzsäure ausgeschüttelt. Nach dem Eindampfen der salzsauren Auszüge bleiben 423 mg Norephedrin-hydrochlorid mit 14,8 Gew.-% (S)-Enantiomerenüberschuss zurück.

### Beispiel 4: Enantiomerentrennung durch ein iteratives Extraktionsverfahren.

Als iteratives Extraktionsverfahren wird Verteilungschromatographie verwendet. Das Chromatographierohr (Durchmesser 3 cm) ist mit einem Stickstoffeinleitungsrohr und mit einem an einen Colora-Kryostat angeschlossenen Kühlmantel versehen. Zur Herstellung der Säule wird eine Suspension von 50 g Kieselgur (Hyflo Super Cel) in 300 ml 1,2-Dichloräthan unter energischem Rühren tropfenweise mit 30 ml einer 1,0-molaren wässrigen NaPF6 Lösung beladen.[Falls das verwendete Kieselgur Eisen enthält, wird dieses während der Chromatographie eluiert. Dieses Eisen, welches die spektrometrischen Bestimmungen stört, lässt sich vor der Verwendung des Kieselgurs durch Waschen mit IN Salzsäure, verdünnter Aethylendiamintetraessigsäure-Lösung und Wasser entfernen]. Die Suspension wird auf einmal in das Chromatographierohr eingegossen, worauf man das Lösungsmittel unter gelindem Stickstoffüberdruck (0,1 bar) abfliessen lässt, bis das Kieselgur gerade noch bedeckt ist. Die so vorbereitete Säule wird zuerst mit 30 ml 1,2-Dichloräthan überschichtet, um das Aufwirbeln des Kieselgurs beim Eintragen des Chromatographiegutes zu verhindern. 100 mg (+)-Norephedrin-hydrochlorid (0,00053 Mol) in 0,5 ml 2,0-molarer wässriger NaPF₆-Lösung werden der Säule in einer Suspension von 1 g Kieselgur in 10 ml 1,2-Dichloräthan zugefügt. Dann lässt man das Lösungsmittel wieder so weit abfliessen, dass das Kieselgur gerade noch bedeckt ist. Die Säule wird mit 300 ml einer 0,3-molaren Lösung von Di-(5-nonyl)-(R,R)-tartrat in 1,2-Dichloräthan überschichtet, worauf man die Kühlung einschaltet. Nach dem Abkühlen des Rohrinhalts auf 0°C (nach etwa 10 Minuten) wird mit der Eluierung begonnen. Die Durchflussgeschwindigkeit beträgt bei einem Stickstoffüberdruck von 0,1 bar 250-300 ml/Std. Es werden Fraktionen von 10 ml aufgefangen und mit einem Spektrophotometer auf ihre optische Dichte bei 263 nm untersucht. Aus den zusammengefügten Fraktionen von 200-270 ml Elutionsvolumen bzw. 330-400 ml Elutionsvolumen bleiben nach dem Aufarbeiten 26 mg (1S)-Noreghedrin-hydrochlorid mit 97,7 Gew.-/ Enantiomerenüberschuss bzw. 27 m^{g} seines (lR)-Enantiomeren mit 97,6 Gew.-% Enantiomerenüberschuss zurück. Beispiel 5; Analog der in Beispiel 4 beschriebenen Arbeitsweise werden die Enantiomeren von Phenylglycin unter folgenden Bedingungen getrennt: Als stationäre Phase werden 40 ml 1-molares Natriumhexafluorphosphat auf 60 g Kieselgel aufgetragen. Als bewegliche Phase dient eine 0,35-molare Lösung von Di-(5-nonyl)-(R,R)-tartrat in 1,2-Dichloräthan. Der Versuch wird bei -10°C durchgeführt. Ausgehend von 100 mg (+)-Phenylglycin-hydrochlorid werden 18,5 mg des praktisch reinen (-)-Enantiomeren (>99 Gew.-%) und weitere 24 mg einer Zwischenfraktion, die 84 Gew.-% des gleichen Enantiomeren enthält, erhalten.

Eine weitere Zwischenfraktion enthält 24 mg eines Gemisches mit 74 Gew.-% des (+)-Enantiomeren. Die Schlussfraktion (21 mg) enthält 92 Gew.-% des (+)-Enantiomeren.

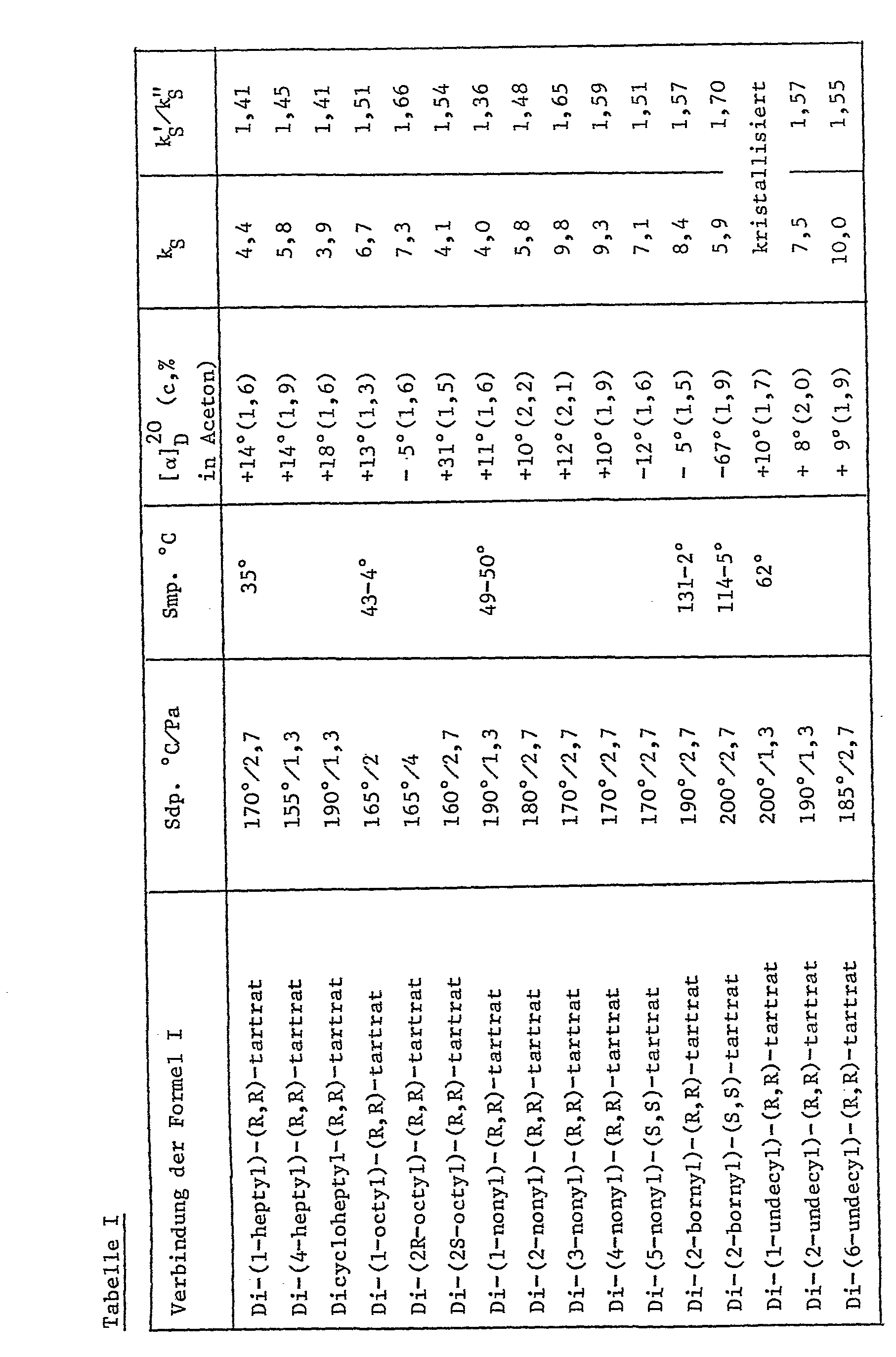

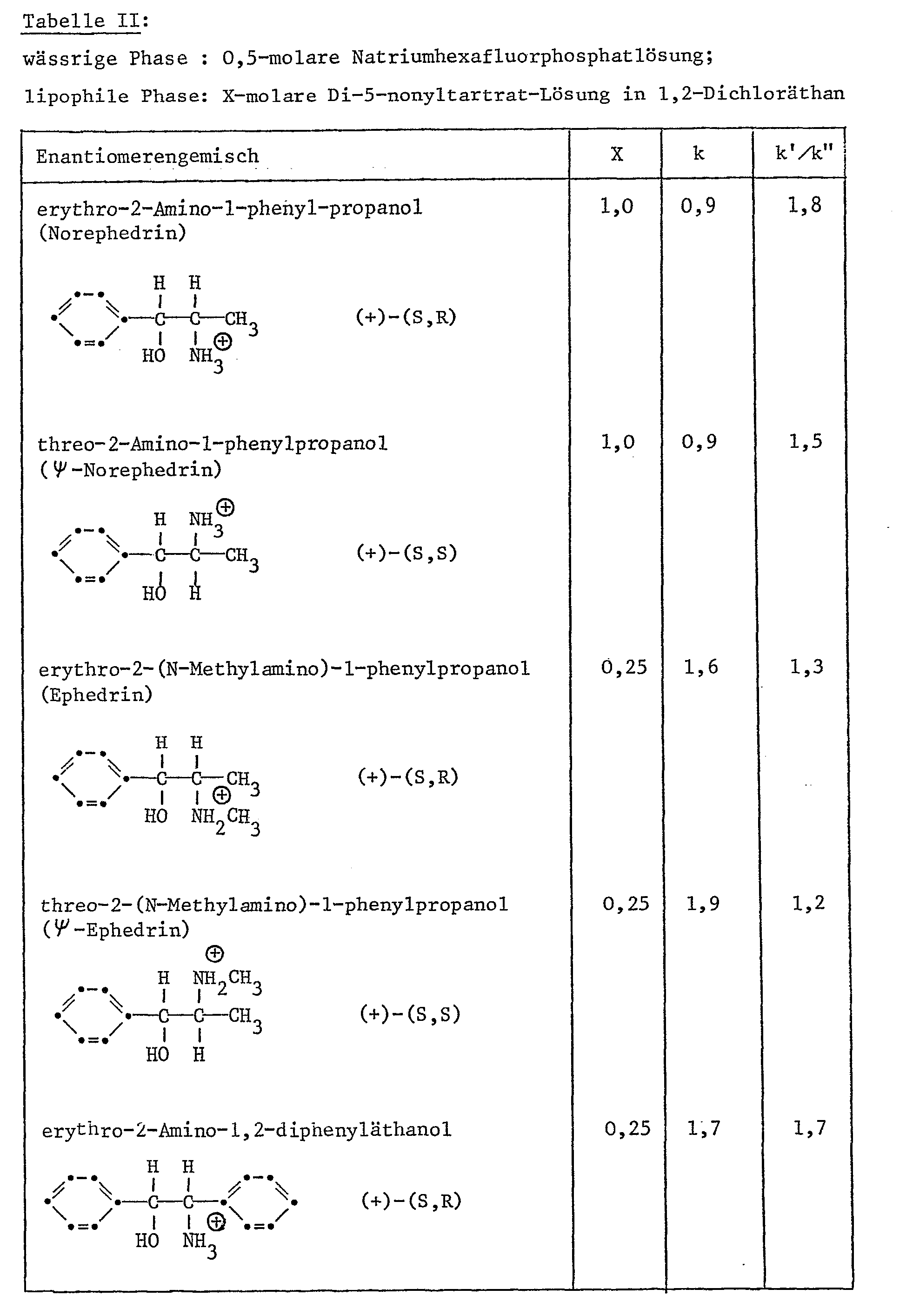

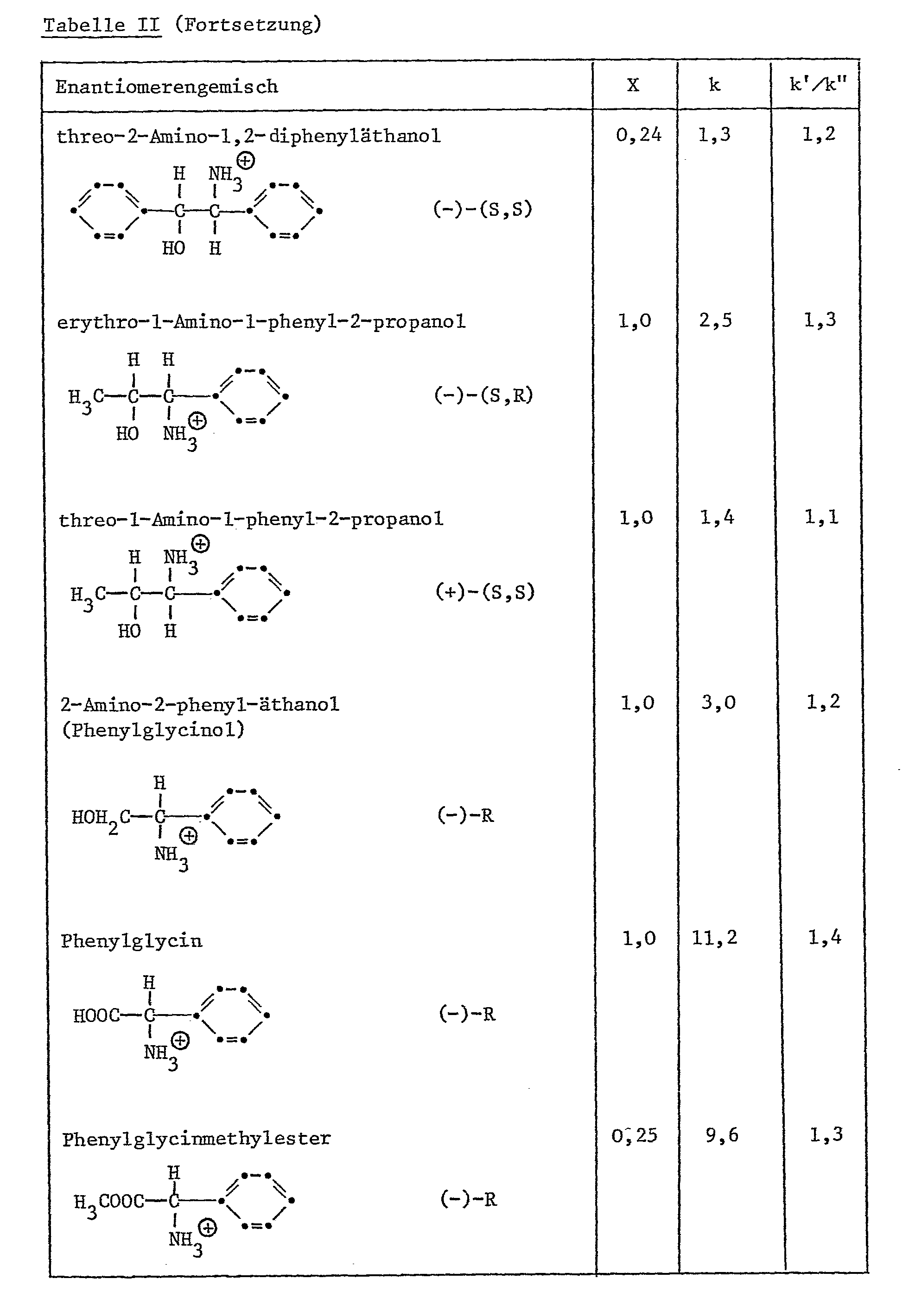

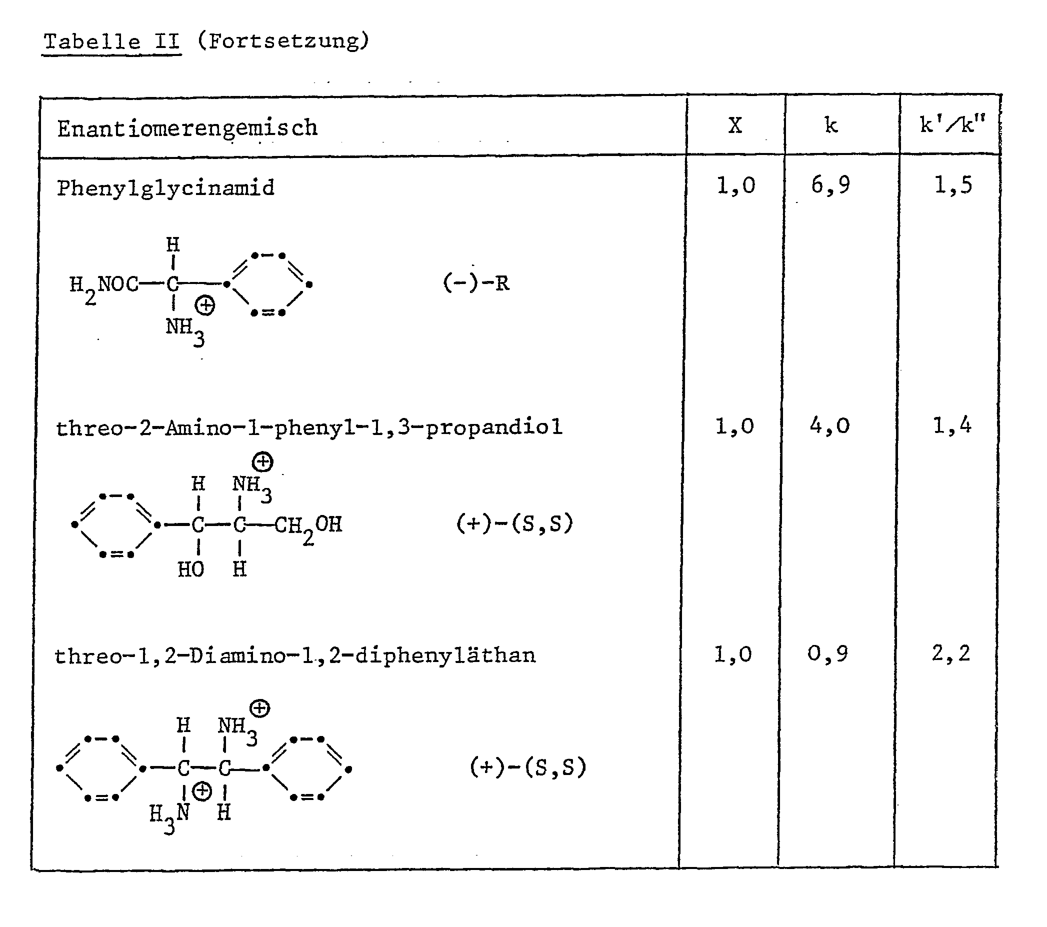

## Patentansprüche

1. Verfahren zur Enantiomeren-Trennung durch Verteilung zwischen flüssigen Phasen, das dadurch gekennzeichnet ist, dass man zwischen einer wässrigen Phase und einer lipophilen Phase, welche eines der beiden optisch aktiven Isomeren eines Weinsäureesters der Formel 1 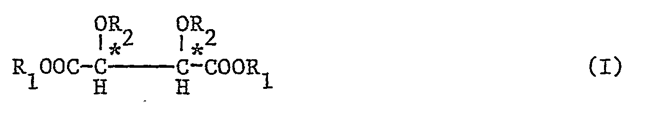 enthält oder aus diesem besteht, ein Enantiomerengemisch einer chiralen Verbindung verteilt, das mindestens teilweise aus einer Verbindung der Formel II 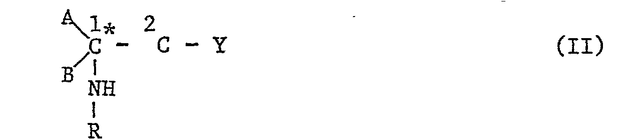 in Form eines Ammoniumsalzes mit einem lipophilen Anion besteht, worin A und B Wasserstoffatome oder organische Reste sind oder A und B gemeinsam mit dem Kohlenstoffatom, an das sie gebunden sind, eine Ringstruktur darstellen, wobei jedoch A und B bzw. die an das ¹C-Atom gebundenen Gruppierungen oder Atome voneinander verschieden sein müssen, sodass die Verbindung chiral ist, R ein Wasserstoffatom oder einen organischen Rest darstellt, Y ein Sauerstoff-, Stickstoff- oder Schwefelatom bedeutet, welches die Ausbildung einer Wasserstoffbrücke ermöglicht, wobei Y über eine Einfach-, Zweifach- oder Dreifachbindung an das Kohlenstoffatom gebunden ist und Y und/oder das Kohlenstoffatom, an welches Y gebunden ist, gegebenenfalls durch Wasserstoff oder organische Reste substituiert sein können, die R gleiche oder verschiedene organische Reste und die R unabhängig voneinander Wasserstoff oder einen organischen Rest bedeuten, wobei das eine der beiden Enantiomeren der zu trennenden chiralen Verbindung in einer grösseren Menge aus der wässrigen Phase in die lipophile Phase übergeht als das andere und somit eine Enantiomerenanreicherung oder Enantiomeren- Trennung erreicht wird.

2. Verfahren nach Anspruch 1, dadurch gekennzeichnet, dass die lipophile Phase zusätzlich zu der Verbindung der Formel I ein mit Wasser nicht mischbares organisches Lösungsmittel enthält, wobei das Lösungsmittel selbst nicht zur Ausbildung von Wasserstoffbrücken befähigt ist.

3. Verfahren nach Anspruch 1, dadurch gekennzeichnet, dass die lipophile Phase zusätzlich zu der Verbindung der Formel I einen Kohlenwasserstoff oder einen halogenierten Kohlenwasserstoff,besonders einen chlorierten aliphatischen Kohlenwasserstoff, enthält.

4. Verfahren nach Anspruch 1, dadurch gekennzeichnet, dass die lipophile Phase zusätzlich zu der Verbindung der Formel I Chloroform, Tetrachlorkohlenstoff, Trichloräthan und insbesondere 1,2-Dichloräthan als Lösungsmittel enthält.

5. Verfahren nach Anspruch 1, dadurch gekennzeichnet, dass in dem in der lipophilen Phase enthaltenen Weinsäureester der Formel I die R₁ aliphatische oder cycloaliphatische Reste sind.

6. Verfahren nach Anspruch 1, dadurch gekennzeichnet, dass in dem in der lipophilen Phase enthaltenen Weinsäureester der Formel I die R₁ Alkyl- oder Alkenylgruppen mit mindestens 4 C-Atomen oder Cycloalkyl- oder Cycloalkenylgruppen mit mindestens 4 C-Atomen sind, die durch Cₗ₋₄-Alkylgruppen substituiert sein können, wobei es sich bei den Cycloalkyl- oder Cycloalkenylgruppen R auch um polycyclische Systeme handeln kann.

7. Verfahren nach Anspruch 1, dadurch gekennzeichnet, dass in dem in der lipophilen Phase enthaltenen Weinsäureester der Formel I die R₁ Alkylgruppen mit 6 - 20 C-Atomen und die R₂ je Methyl oder Wasserstoff darstellen.

8. Verfahren nach Anspruch 1, dadurch gekennzeichnet, dass in dem in der lipophilen Phase enthaltenen Weinsäureester der Formel I die R₁ gegebenenfalls durch eine Methylgruppe substituierte mono- oder bicyclische Cycloalkylgruppen mit insgesamt 6 - 20 C-Atomen und die ^{R}₂ je Methyl oder Wasserstoff darstellen.

9. Verfahren nach Anspruch 1, dadurch gekennzeichnet, dass in dem in der lipophilen Phase enthaltenen Weinsäureester der Formel I die R₁ je die Cycloheptyl- oder 2-Bornylgruppe und die R₂ je Wasserstoff bedeuten.

10. Verfahren nach Anspruch 1, dadurch gekennzeichnet, dass in dem in der lipophilen Phase enthaltenen Weinsäureester der Formel I die R₁ je die 5-Nonylgruppe und die R₂ je Wasserstoff bedeuten.

11. Verfahren nach Anspruch 1, dadurch gekennzeichnet, dass die zu trennende chirale Verbindung ein a-Aminoalkohol, eine a-Aminosäure, ein a-Aminosäureester oder ein a-Aminosäureamid ist.

12. Verfahren nach Anspruch 1, dadurch gekennzeichnet, dass die zu trennende chirale Verbindung einEnantiomerengemisch von Ephedrin, Pseudo-Ephedrin, Norephedrin, Pseudo-Norephedrin, erythro-2-Amino-1,2-diphenyläthanol, threo-2-Amino-1,3-diphenyläthanol, erythro- oder threo-l-Amino-l-phenyl-2-propanol, Phenylglycinol, Phenylglycin, Phenylglycinmethylester, Phenylglycinamid, threo-2-Amino-l-phenyl-1,3-propandiol oder threo-1,2-Diamino-1,2-diphenyläthan ist.

13. Verfahren nach Anspruch 1, dadurch gekennzeichnet, dass die zu trennende chirale Verbindung ein Enantiomerengemisch von Norephedrin, Ephedrin, Pseudo-Ephedrin, threo-2-Amino-1-phenyl-1,3-propandiol, Phenylglycin, Phenylglycinmethylester oder Phenylglycinamid ist.

14. Verfahren nach Anspruch 1, dadurch gekennzeichnet, dass das lipophile Anion Hexafluorphosphat, Hexafluorsilikat, Hexafluorantimonat, Hexafluorarsenat, Tetraphenylborat, Tetrafluorborat, Pikrat, Perchlorat, Rhodanid oder Jodid ist.

15. Verfahren nach Anspruch 1, dadurch gekennzeichnet, dass das lipophile Anion Tetraphenylborat oder Tetrafluorborat ist.

16. Verfahren nach Anspruch 1, dadurch gekennzeichnet, dass das lipophile Anion Hexafluorphosphat ist.

17. Verfahren nach Anspruch 1, dadurch gekennzeichnet, dass man das Trennverfahren bei einer Temperatur durchführt, die oberhalb des Erstarrungspunktes der wässrigen Phase bzw. der lipophilen Phase liegt.

18. Verfahren nach Anspruch 1, dadurch gekennzeichnet, dass man das Trennverfahren bei einer Temperatur im Bereich von-5 bis +5°C durchführt.

19. Verfahren nach Anspruch 1, dadurch gekennzeichnet, dass man die Enantiomerentrennung durch iterative Verteilung des Enantiomerengemisches zwischen der wässrigen und lipophilen Phase vornimmt.
